# EUROPEAN PATENT APPLICATION

(11) **EP 2 535 084 A1**
(43) Date of publication of application: **19.12.2012**
(21) Application number: 11741926.7
(22) Date of filing: 09.02.2011
(51) Int. Cl.: A61N 2/02

(54) **PORTABLE DIGITAL TRANSDUCER DEVICE THAT IS PROGRAMMABLE, HAS HIGH DISCRIMINATION AT LOW FREQUENCY AND LOW INTENSITY**

(30) Priority: 10.02.2010 ES 201030183
(71) Applicant: Pneuma Research, S.L., 41004 Sevilla (ES)
(72) Inventor: MAESTU UNTURBE, Ceferino, E-28690 Brunete (Madrid) (ES); MIKUSKI, Frank, E-28007 Madrid (ES); DEL POZO GUERRERO, Francisco, E-28223 Pozuelo de Alarcón (Madrid) (ES)
(74) Representative: Carvajal y Urquijo, Isabel
(86) International application number: PCT/ES2011/000025
(87) International publication number: WO 2011/098638

(57) **Abstract**

The invention relates to a, portable digital transducer device that is programmable, has high discrimination at low frequency and low intensity, comprising a modular, portable digital device that generates electromagnetic fields and can be used for many medical applications. The device can be programmed using software in terms of the main parameters thereof (amplitude, waveform, frequency, pulse sequence, positive-negative model, etc.). In addition, the device is completely portable, [lightweight and powered by rechargeable batteries. The high degree of modularity of the device allows flexibility in terms of future developments in accordance with new required parameters. The device also enables the synchronous construction of generator arrays for linear and sequential applications. The device has high discrimination at frequencies below 1 milli-Hz and an intensity of 0.001 µA.

## Description

### Object of the Invention

The present invention relates to a modular, portable digital device that generates electromagnetic fields and can be used for many medical applications. The advantage of this new design is the miniaturization of its circuits with respect to those already existing in the market, which further allows it to be programmable using software in terms of the main parameters thereof (amplitude, waveform, frequency, pulse sequence, positive-negative model, etc.). In addition, the device is completely portable, lightweight (less than 100gr) and powered by rechargeable batteries. The high grade of modularity of the device allows it to be flexible in future developments according to new required parameters. The device also enables the synchronous construction of generator arrays for linear and sequential applications.

This invention described below consists of a pulsating field generator system that is programmable in different wave forms, with digital control. It has a high discrimination capacity at a frequency less than one thousandth of a Hz and 0.001 pA intensity. It is therefore a device which by its application range (within the ICNIRP regulations, CENELEC on exposure to magnetic fields) allows it to be used as a therapeutic unit in different medical treatments.

Consequently, the device is within the field of biomedical applications in the low intensity and low frequency transcranial magnetic stimulation section in the framework of biomedical engineering.

### Background of the Invention

Magnetic transcranial stimulation is a non-invasive method for stimulating cerebral activity; it was first used in humans in the 80s, where fast changes in external magnetic fields induce Eddy currents in the conductive tissue of the human brain, allowing the non-invasive manipulation of neural activity both at the microscale and the macroscale level. This activity has a potential capacity for therapeutic action for treating disorders related with the so-called central pain, a product of frequency alteration in coded neural networks.

It has been acknowledged for more than one hundred years that electricity and magnetism are interdependent units, linked by means of Maxwell equations (Bohning 2000), a current passing through a conductor generates a magnetic field perpendicular to the direction of the current. In the last few years a growing number of reports have appeared, demonstrating significant effects of extremely low frequency electromagnetic fields of (ELF,EMF) on different aspects of animal and human behaviour. The reports have further shown that exposure to ELF fields affects cerebral electrical activity. This system is non-invasive since it will use low intensity pulsating magnetic fields (under CENELEC regulations and ICNIRP recommendations). They were first used in humans in the 80s, where the controlled pulse magnetic field allowed inducing Eddy currents into nerve tissue, which allows modifying the electrical properties of the underlying tissues, with a broad therapeutic potential.

The systems developed to date use high intensity fields (TMS). Some investigations allow assuming that low intensity and low frequency electromagnetic fields are capable of modifying cerebral functions and low intensity generator devices have been developed to that end, fundamentally those developed by Jacobson enterprises Inc, generators for repairing bone fractures and muscular dysfunctions, based on field generator systems with external analogue-digital modulators and not integrated in a single chip. Earthpulse TM systems are also known where their programmable systems do not achieve suitable frequency precision.

In patent US005014699 the described device has a small generator producing a 0-5 V signal by means of elemental pulses of 2-10 ms using symmetrical sinusoidal and triangular waveforms with an interval of 60-65 ms between pulses but using high intensities, so it differs from the present' development in intensity and precision, furthermore, it is not a programmable device. Patents WO94/13357 and WO0078267A2 describe a generator producing high modulated frequencies at low frequency giving rise to a rotating magnetic field. The design and the applications differ significantly from the present development. In turn, patent EP 0048451A1 describes a capacitor discharging circuit for discharging through a resistor, a system regulating the current pulse and the space between pulses. In this case, it is a device which is neither programmable, nor portable. Like in the present invention, the Jacobson developments use very weak magnetic fields, around 10⁻²⁰ Gauss, applied through a Hemholtz coil. It is neither a programmable nor portable device even though fields of a range similar to those of the present case are used. Another similar device is that described in patent WO0078267A2, which has a portable system for applying magnetotherapy such as TENS, i.e., by inducing an electric field from a magnetic field. To that end it needs a lot of power, is not programmable, and rather imprecise in frequency.

The applicant is unaware of portable modular digital magnetic actuators of high precision in frequency and amplitude applicable to highly effective and safe medical treatments.

### Description of the Invention

The portability of the system and the supply capacity in duration and disconnection from the electrical grid allow this modular system to have a large working capacity, differentiating it from the systems found on the market.

To that end and more specifically, the device of the invention is made as small portable electronic equipment powered by rechargeable or primary batteries, designed for generating low intensity electromagnetic signals. These signals can be programmed in different ways, frequencies, magnitude and duration using to that end an external programming system. The device enables being applied to an intermediate number of actuators, since it behaves as a current generator regardless of the load it has to withstand.

The design of the equipment allows great precision in the area of ELF frequency, with a resolution of thousandths of a Hz and very low intensities of 0 1 mA but having the high precision necessary for medical applications in the area of magnetic stimulation.

More specifically, the following six fundamental elements are included the device of the invention:
- Intelligent battery charging circuit.
- Power supply circuit.
- Digital circuit.
- Analogue circuit.
- USB connection and protection circuit.
- Liquid crystal display.

The device can be supplied by two types of battery, one based on lithium ion polymer batteries or likewise lithium ion batteries. The other type of battery which can be used in this device is based on primary batteries, formed by two lithium batteries of 3 VDC voltage. The latter can be used on those occasions when the internal lithium ion battery has run out and the equipment is to be used.

The intelligent charging circuit includes intelligent electronics monitoring the initial charging voltage of the batteries and according to it, establishes a charging program designed for charging the battery in the least amount of time possible causing the least amount of wear possible to the internal chemistry of the batteries subjected to charging.

This circuit includes a mini power jack input connector widely used in cellular telephones designed for using a wall transformer charger of 5VDC at 800 milliamperes. Likewise, the intelligent charging circuit can withstand up to 12 volt voltages and up to one ampere.

This circuit likewise includes a dual-colour LED whereby the state of charge of the battery can be known, indicating in red that the circuit is in the process of charging and in green when the charging is complete and the charger can be disconnected from the equipment.

A circuit formed by two Schottky rectifiers is included to be able to use the two types of batteries, the internal battery and lithium batteries, which rectifiers allow protecting the equipment from polarity reversal in the event that the lithium batteries are incorrectly installed and both types of battery can be used without damaging the supply circuit.

Therefore, and in relation to the state of the art, the transducer system proposed by the invention modifies the supply system using batteries having a greater capacity, i.e., lithium ion/lithium ion polymer batteries because acid/lead batteries must not be used in medical equipment and especially in equipment which must be manipulated close to patients or people since this type of battery tends to internally generate hydrogen gas in short circuit conditions which can cause an explosion, break the case or activate the safety valve with the corresponding acid electrolyte spillage. Therefore, this new type of supply was incorporated due to its safety features, weight and physical size. The use of lithium ion/lithium ion polymer batteries is based on the fact that the latter have a much lower weight and cost in reference to both the batteries themselves and the charging equipment.

A second differentiating feature is the microcontroller; in other models two low processing capacity microcontrollers are used, one for managing the generation of electrical signals typical of the stimulating equipment and the other for managing the series port. A serial to USB converter had to be added externally to the equipment in this latter case in order to program the equipment using an external computer.

In the case of the microcontroller of the present invention, it incorporates a large processing capacity single controller with ARM technology, including a digital-analogue converter (DAC) internally providing 60 times more precision in the generated analogue signals, especially in those signals different from the square form. The fact that the DAC is located inside the microcontroller eliminates external noises and improves the quality of the generated signals with the corresponding equipment cost reduction. This microcontroller also includes the software management and the electronics necessary for generating a USB 2.0 port eliminating the use of external serial to USB converters. In addition to managing the USB port and signal generation, the new microcontroller manages the handling of the rest of the equipment as in the case of signaling control, +/-5V internal power supplies, the buzzer also leaving enough free ports for incorporating an LCD display or any other attachment in the future. This new device further has an internal DAC. The placement of an external DAC implies using a parallel port necessary for handling this device as well as higher costs. In the proposed model, the DAC is located inside the silicon of the microcontroller and therefore does not take up any port except the signal output port; it consumes less power, therefore achieving that the internal batteries of the equipment last longer and makes the latter less vulnerable to external noises. The new device includes an electronic circuit for processing the analogue signals, the circuit uses a single integrated circuit (operational) instead of two (typically used). This reduces power consumption, providing greater working autonomy in the possible medical applications.

Likewise, an input with a voltage of 3.3 volts is used in the new equipment. In this sense two power supplies necessary for operating the part of the analogue processing of the signals generated by the DAC had to be included. Likewise, two +/-5V DC/DC sources with chips and connected ultra low consumption electronics were designed. Physical space for placing two lithium batteries of 3V each, whereby the equipment can be supplied for more than 40 hours if power from the grid is not available for recharging the internal batteries was included as an additional element in the new equipment. This allows performing field work or work where there is no electrical grid.

### Description of the Drawings

To complement the description which is being made and for the purpose of aiding to better understand the features of the invention according to a preferred practical embodiment thereof, a set of drawings is attached as an integral part of said description where the following has been depicted following with an illustrative and non-limiting character:
Figure 1 shows a block diagram of the different essential elements included in a portable digital transducer device carried out according to the object of the present invention.

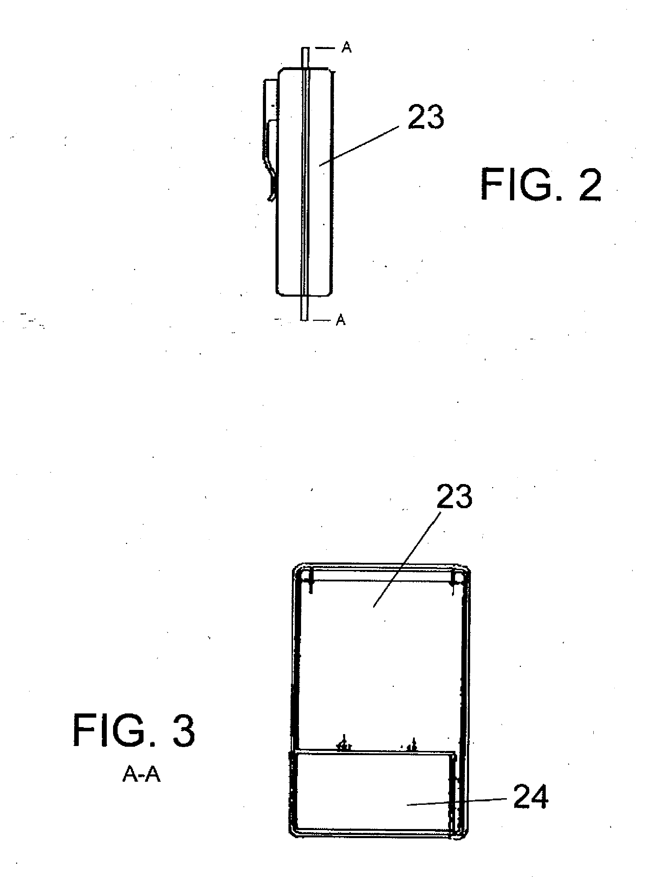

Figure 2 shows a profile view of the case housing the device of the preceding figure.
Figure 3 shows a cross-section view according to section line A- A of Figure 2.
Figure 4 shows a detailed diagram of the digital circuit included in the circuit of Figure 1.
Figure 5 shows a detailed diagram of the intelligent battery charging circuit included in the circuit of Figure 1.
Figure 6 shows a diagram of the linear regulator circuit included in the circuit of Figure 5.
Figure 7 shows a detailed diagram of the analogue circuit or current stage included in the circuit of Figure 1.
Finally, Figures 8 and 9 show respective detailed diagrams of the power supply and protection circuits included in the circuit of Figure 1.

### Preferred Embodiment of the Invention

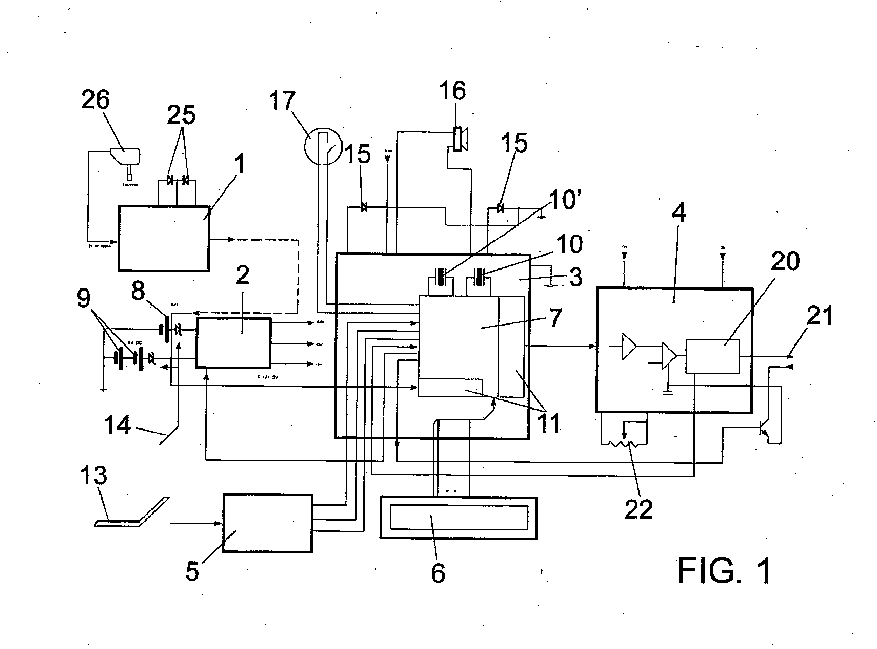

In view of the described figures and especially Figure 1, it can be observed how six fundamental elements, interrelated to one another, made as an intelligent battery charging circuit (1), a power supply circuit (2), a digital circuit (3), an analogue circuit (4), a USB connection and protection circuit (5), and a liquid crystal display (6) are included in the device of the invention.

In terms of the power supply circuit (2), it allows generating the three working voltages necessary for device operation. The digital circuit (3) needs a 3.3 VDC working voltage and the analogue circuit (4) necessary for processing the signals produced by the equipment requires two working voltages, one being +5 VDC and the other -5 VDC.

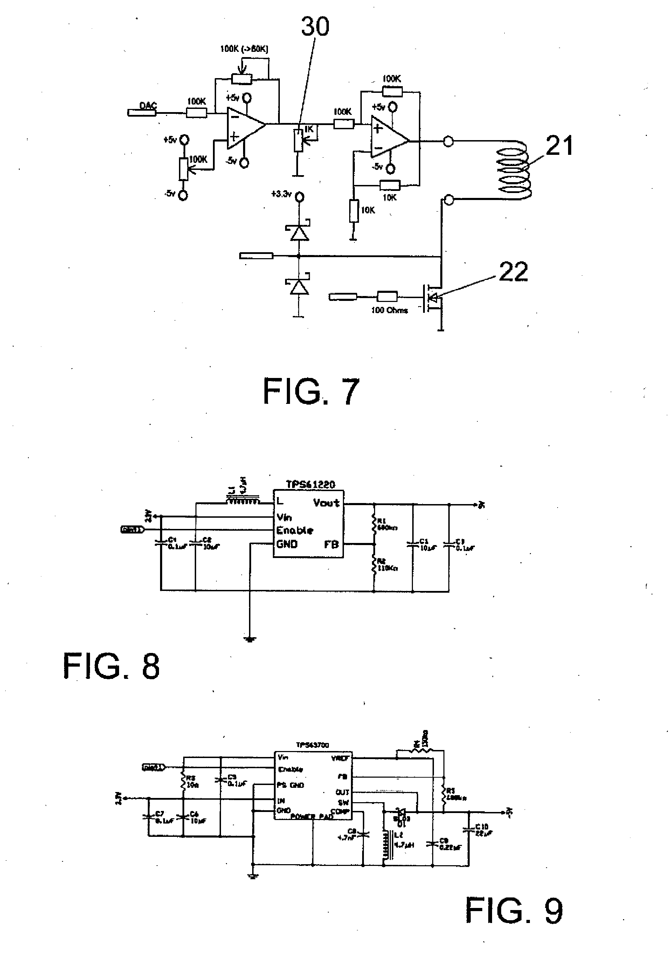

The 3.3 V supply voltage is obtained using an LDO or fixed-voltage regulator of 3.3 volts. On the other hand, the +/- 5 volt supply voltages are generated inside the equipment using two different power supplies including respective integrated circuits, which are shown in detail in Figures 7 and 8.

The two 5 volt power supplies include a circuit allowing them to be turned on or turned off using an I/O of the microcontroller (7). This feature was designed for saving power when the equipment is on.

In this sense, this power supply circuit can operate using both the 3.7-4 VDC voltage from the internal rechargeable battery (8) or using the 6 volt voltage from the two lithium batteries (9) of 3 volts each which are arranged in series in the case of the equipment.

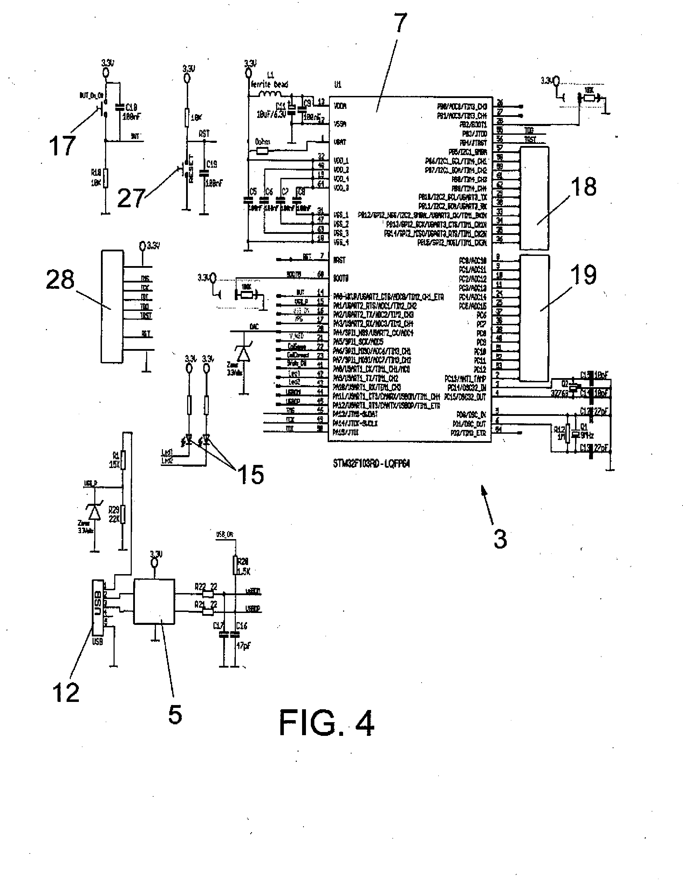

In turn, the digital circuit (3) shown in detail in Figure 4 is formed by an ARM microcontroller (7) which has two oscillator circuits (10-10'), one of them used for normally operation of the equipment, operating with a crystal with an 8 Mhz frequency. The other optional oscillator circuit is formed by a 32.768 Khz low frequency crystal which could be used in applications which require carrying out a precise monitoring of time with deviations of less than a couple of seconds per year.

The oscillator circuit of the microcontroller has an internal PLL circuit which allows using the 8 Mhz reference oscillator circuit for multiplying the operating frequency of the processor at frequencies of more than 70 Mhz, thus allowing this microcontroller to generate analogue signals that are very precise over time.

The microcontroller internally includes a digital-analogue converter circuit (11) which allows generating any type of wave signals referenced to 0 volts. In this sense the equipment includes internal software which allows generating several types of signals such as:
Square signals.
Sinusoidal signals.
Saw signals.
Triangular signals.

By including the suitable software, the digital-analogue converter (11) can generate any type of complex signal as needed to be used according to the pathologies which will be necessary to treat in the future.

The microcontroller (7) internally includes a pair of, ports (18-19) and the firmware necessary for implementing a USB 2.0 connection (12). This allows a USB connection to be included for connecting to any type of external computer (13) necessary for being able to program the type of treatment necessary for treating different types of pathologies according to the individual needs of each patient. This circuit allows not having to use USB-serial converters for the purpose of being able to program the different types of treatments.

The analogue-digital converter (11) is internally included in the microcontroller, this, together with the protection circuit (5) and a voltage splitter (14), which are used for the purpose of being able to detect the working voltage of the batteries, for the purpose of detecting low battery states and thus being able to indicate to the user that the equipment will stop producing signals caused by a low battery state effect.

The digital circuit additionally includes control for the human machine interface formed by the following devices:
- LED indicators (15).
- Buzzer or noise or tone generator (16).
- Liquid crystal display (6).
- On/off button of the equipment (17).
- Detection device for detecting a connection of an external computer to the USB port (12).
- Device for activating and disconnecting the signal generating circuit.
- Detection device for detecting the opening of the magnetic signal generating circuit.

The LED indicators (15) together with the buzzer or tone generator (16) allow knowing the operating state of the device.

The digital circuit (3) also includes a port which allows connecting the liquid crystal display (6) to the microcontroller if such device is necessary.

This digital circuit (3) also includes a multifunction button whereby the device can be turned off and on and treatment can be activated and deactivated.

As well as the digital circuit, it includes a digital-analogue converter (11) for generating the different programmed wave signals referenced to 0 volts, the analogue circuit (4) transforms the signals received in the form of a 0-3.3 volt voltage into +/-5 volt NRO (Non Return to zero) signals converted into current signals with a maximum capacity of 1 milliampere. The signals converted into current by this circuit can also be set to a lower intensity by means of setting the software reducing the amplitude of the different generated signals in a digital-analogue converter which can also change the frequency and the working cycle of the generated signals.

In addition to these aforementioned functions, this circuit includes a continuity detecting circuit (20) keeping the microcontroller (7) informed about whether or not the coils (21) are connected to the equipment in a manner such that the working session can or cannot be started.

The analogue circuit (4) additionally includes an offset setting circuit (22) as a way of centrally adjusting the current signals and for adjusting them with the correct working cycle. This adjustment is only performed once at the time of manufacturing each device and therefore it is not available, neither to the operator nor the user of the equipment.

In turn, the USB connection and protection circuit (5) is responsible for protecting the microcontroller (7) from static electricity which is generated in the environment and which usually damages microcontrollers when such high voltage directly contacts the ports thereof. This circuit includes an integrated static protector, a filter circuit for the differential connection typical of USB ports and a wiring detection circuit which allows the micro to know when a computer has been connected.

In terms of the liquid crystal display (6), it is an optional device allowing the doctor or the staff operating the device to know the type of programming and application time which is being used in each working section, such information is only shown while the equipment is connected to a USB port, in a manner such that the programming defined in the equipment is the correct one being able be to be confirmed. This device can also be used for showing the users of the equipment when the session has ended.

The described circuitry will be housed in a case or casing (23) made from non-ferromagnetic material, said circuitry being integrated on a printed circuit board or PCB.

The equipment will be supplied, as mentioned above, through a rechargeable lithium ion or lithium ion polymer battery, having a wire harness and battery contacts for wiring of the electronics board with the primary lithium batteries. The latter can be used for activating the equipment in those cases where the internal battery is uncharged.

Likewise, it will include low level software which has the capacity of programming the microcontroller (3) of the equipment so that it can operate with the functionalities with which the equipment was designed, generating electrical signals of different characteristics and carrying out the entire automatic control of the system.

Additionally, it will be complemented with high level software which is installed in any laptop or PC for being able to programme the type of indicated application specific for each pathology using to that end a USB connection.

The casing (23) will preferably be made from plastic, a special compartment (24) is included for housing a pair of primary lithiu,m batteries (9) of 3V each which are placed according to the instructions printed on said case.

Likewise, it will include two LED indicators (15), a red LED and another green LED, on its upper front part whereby the type of programme and operation of the device is indicated.

It also includes a dual-colour LED indicator (25) on its side part which allows knowing the state of charge of the rechargeable internal battery when it is in the process of charging.

Together with the LED charge indicator the equipment includes a hole where the connector of the charger (26) is installed. The connector is a mini connector of 2 millimetres in diameter. The characteristics of specified charge supply in the order of 800mA at 5VDC.

The Mini USB connector (12) whereby the equipment is programmed is located at the other end or on the right side of the box.

The push button (17) which allows turning the equipment on and off when treatment is to be performed is located on the upper left side of the case. The programmed treatment can be started or ended using this same button with a specific type of sequence.

A female SMA connector used for screwing the male SMA connector used by the applicator is located on the upper right face of the case.

The four-layer PCB board is designed with electronic SMD components and passive elements with predominant SMD 0603 technology.

Returning to the digital circuit (3), it consists of a Cortex microcontroller (7), model SMT32F103 including a reference crystal of 8 MHz and another of 32.768KHz of optional use.

Pin No. 20 of the microcontroller is a DAC or digital-analogue signal conversion output. The electrical signals defined at the start as programmable signals which can be of four types, square, triangular, sinusoidal or ramp will be generated at this point. These signals are generated by means of low level software housed in the non-volatile memory of this microcontroller.

This same micro controls the turning on and off of the power supplies, the detection of the existence of the connection with the stimulating helmet, detection of battery charge (8-9), automatic connection of the coils (21) of the stimulating helmet in order to enable programming sessions lasting for a specific amount of time, the control of the green and red operation LEDs (15), handling the USB port (12) for programming the type of treatment using an external computer (13), the turning on and off of the device among other things.

It includes electrostatic protection for the USB port, an internal reset button (27) if necessary, a connector (28) with the signals of the JTAG or programming port of the microcontroller for updating the low level software and another three jack type connectors are also included whereby other ports or output and input of the microcontroller used can be programmed for the purpose of adding liquid crystal devices and manual control push buttons.

The PCB of the device also includes an electronic circuit and USB connector with a 5 volt electrostatic protection and detection circuit (5) typical of the USB wiring, provided by any computer whether portable or PC.

Going on to analyse the intelligent battery charging circuit (1), and in order to extend the service life of the batteries and to enable recharging their power content in the shortest time possible, an intelligent battery charging system was designed monitoring the external charge voltage, the internal voltages generated by the chemical agents and way of charging of each battery in particular, this is such that an optimal charge programming can be made without deteriorating the internal chemistry thereof.

This device was designed to be able to use a charger (26) as a charging element. The charger must be used with a charging voltage of between 5 and 12 volts and maximum charging capacity of 800 milliamperes with a DC output. On the other hand, the mini Jack power charging connector or plug with a diameter of 2 mm and with positive voltage in the center of said connector.

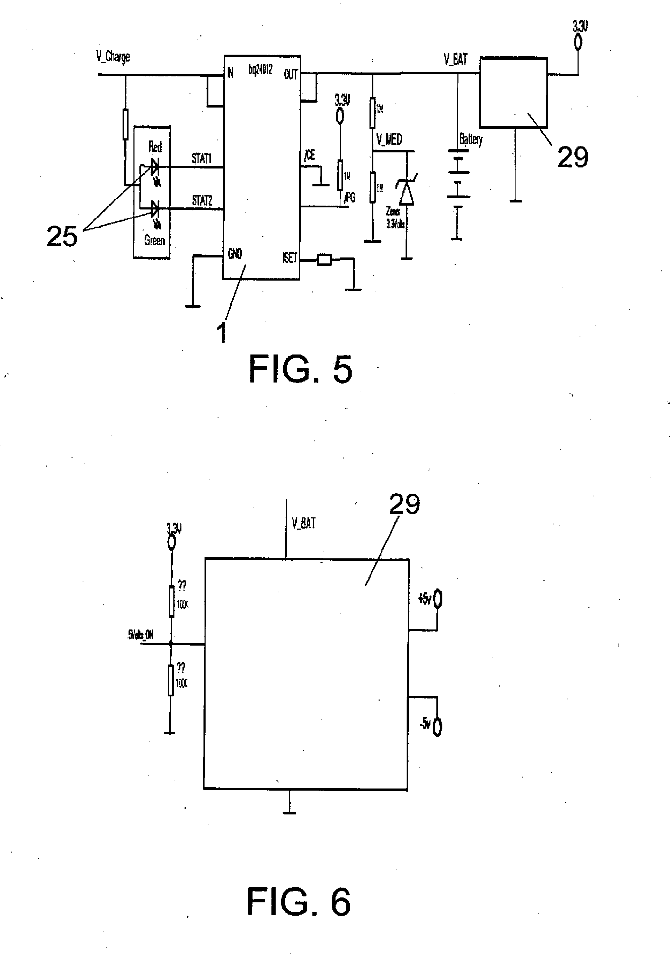

To that end, the intelligent charging circuit (1) shown in detail in Figure 5 has been included in the PCB, which is based on a Texas Instrument BQ24012 circuit or chip which is designed for that purpose, having a low dropout linear regulator (29), shown in detail in Figure 6.

Returning to the analogue circuit (4), it is designed for treating the electrical analogue signals, generated by the microcontroller for converting them into current signals which will be converted into magnetic waves of different shape, frequency and magnitude in the coils (21) of the applicator.

This current amplifier is supplied with a positive voltage and another negative voltage of 5 volts not connected to ground, including an output towards the female SMA connector where the stimulating helmet is plugged in, the analogue signal output from the DAC of the microcontroller in its pin no. 20. On the other hand an output signal is implemented in this circuit towards the microcontroller indicating that the stimulating helmet is connected and lastly an output of the microcontroller for activating the electric circuit generating the signals towards the stimulating helmet by means of an N channel Mosfet transistor.

A potentiometer (30) necessary for adjusting the turning off of the generated current signal since the gain and current adjustment are fixed at the time of assembling the PCB is included in this electronic circuit.

As has been mentioned above, the equipment includes three power supplies and a protection circuit. The main power supply has been designed for providing the voltage of 3.3 volts DC, whereby the microcontroller and the rest of the electronic components forming the electronic system of the device are supplied. This with the exception of the circuit of current stage which is supplied with +/- 5 volts.

The 3.3 volt source uses a Texas LDO voltage regulator, model REGI 13-3.3 with a direct input of the lithium ion or lithium ion polymer battery the working voltage of which ranges between 3.3V and 4 volts. This electronic circuit also supplies the 3.3 volt voltage whereby it is stabilised.

The 3.3 volt source uses a Texas LDO voltage regulator, model REGI 13-3.3 with a direct input of the lithium ion or lithium ion polymer battery the working voltage of which ranges between 3.3V and 4 volts. This electronic circuit also supplies 3.3 volt voltage whereby the two power supply circuits of plus and minus 5 volts are supplied.

The 3.3 volt supply circuit can also be supplied from external batteries and therefore a protection circuit formed by Schottky diode has been included to prevent voltage reversal from the lithium ion/lithium ion polymer batteries. Two primary lithium batteries which generate a potential of 3 V volts each are used in this external battery supply. These two batteries are connected in series inside the case of the equipment for generating a 6 volt voltage whereby also supplying the LDO voltage regulator described above.

Two 5 volt power supplies, one being positive voltage the other being negative, are included in the printed circuit to supply the current amplifier of the stimulator.

The positive 5 volt power supply is designed with a DC/DC integrated circuit model TPS 61220 the layout of which is shown in the preceding drawing.

The negative 5 volt source included in the printed circuit of the stimulator is designed with an Inverter integrated circuit model TPS63700 the layout of which of shown in the preceding drawing.

Both 5 volt power supplies are controlled by means of the microcontroller (7) which through pin 41 thereof can turn the operation thereof off and on.

The electronic circuit responsible for recharging the internal batteries is designed for charging in an intelligent manner both lithium ion batteries and lithium ion polymer batteries.

In the case of this equipment, any of these batteries the physical dimensions of which allow installation thereof in the plastic case can be used. In the case of this equipment, batteries with a physical dimension of 40x60x6mm and with a charging capacity greater than 850 milliamperes thereby allowing operating autonomy of the equipment exceeding 30 hours have been used.

The other type of rechargeable battery which can be used in this equipment is a pyramidal lithium ion battery commonly used in cellular mobile telephony, the charging features of which are similar to those discussed above in reference to lithium ion polymer.

Lastly, the equipment can be supplied with primary lithium AA batteries.

The equipment can be used by means of two primary lithium batteries which have a power store of about 1.20 amperes and a working voltage of 3 volts if a battery charger is not available. If this type of supply is used two batteries connected in series are required, which allows the equipment to operate for 50 hours.

## Claims

1. A programmable, portable digital transducer device with high discrimination at low frequency and low intensity, **characterised in that** it is formed from a small portable electronic equipment, powered by rechargeable or primary batteries (8-9), wherein there are established an intelligent battery charging circuit (1), a power supply circuit (2), a digital circuit (3), an analogue circuit (4), a USB connection and protection circuit (5), and optionally a liquid crystal display (6), having been provided that the power supply circuit incorporates generating means for generating three working voltages, one for the digital circuit (3), and two for the analogue circuit (4), while the digital circuit (3) is formed by an ARM microcontroller (7) which has at least one oscillator circuit (10-10'), preferably two, internally including a digital-analogue converter circuit (11) which allows generating any type of wave signals referenced to 0 volts through complementary programming software.

2. The programmable, portable digital transducer device with high discrimination at low frequency and low intensity according to claim 1, **characterised in that** the supply voltage of the digital circuit (3) is obtained using an LDO or fixed-voltage regulator, while the supply voltages of the analogue circuit are generated inside the equipment using two different power supplies including respective integrated circuits.

3. The programmable, portable digital transducer device with high discrimination at low frequency and low intensity according to claim 1, **characterised in that** the two power supplies of the analogue circuit include a circuit allowing them to be turned on or turned off using an I/O of the microcontroller (7).

4. The programmable, portable digital transducer device with high discrimination at low frequency and low intensity according to claim 1, **characterised in that** the microcontroller (7) internally includes a pair of ports (18-19) and the firmware necessary for implementing a USB 2.0 connection (12).

5. The programmable, portable digital transducer device with high discrimination at low frequency and low intensity according to claim 1, **characterised in that** the analogue-digital converter (11) is included inside the microcontroller together with the USB connection and protection circuit (5) and a voltage splitter (14).

6. The programmable, portable digital transducer device with high discrimination at low frequency and low intensity according to claim 1, **characterised in that** the digital circuit includes control for the human machine interface consisting of the following devices:
- LED indicators (15).
- Buzzer or noise or tone generator (16).
- Liquid crystal display (6).
- On/off button of the equipment (17).
- Detection device for detecting a connection of an external computer to the USB port (12).
- Device for activating and disconnecting the signal generating circuit.
- Detection device for detecting the opening of the magnetic signal generating circuit.

7. The programmable, portable digital transducer device with high discrimination at low frequency and low intensity according to claim 1, **characterised in that** the digital circuit (3) includes a port which allows connecting the liquid crystal display (6) to the microcontroller if such device is necessary.

8. The programmable, portable digital transducer device with high discrimination at low frequency and low intensity according to claim 1, **characterised in that** the digital circuit (3) includes a multifunction button whereby the device can be turned on and off, and the treatment can be activated and deactivated.

9. The programmable, portable digital transducer device with high discrimination at low frequency and low intensity according to claim 1, **characterised in that** the analogue circuit (4) incorporates means for transforming the signals received in the form of voltage into NRO (Non Return to zero) signals converted into current signals with a maximum capacity of 1 milliampere, being able to be additionally set to a lower intensity by means of a software setting reducing the amplitude of the different generated signals in the digital-analogue converter which can also change the frequency and the working cycle of the generated signals.

10. The programmable, portable digital transducer device with high discrimination at low frequency and low intensity according to claim 1, **characterised in that** the analogue circuit (4) includes a continuity detecting circuit (20) keeping the microcontroller (7) informed about whether or not the coils (21) are connected to the equipment in a manner such that the working session can or cannot be started.

11. The programmable, portable digital transducer device with high discrimination at low frequency and low intensity according to claim 1, **characterised in that** the analogue circuit (4) includes an offset setting circuit (22).

12. The programmable, portable digital transducer device with high discrimination at low frequency and low intensity according to claim 1, **characterised in that** the USB connection and protection circuit (5) includes an integrated static protector, a filter circuit for the differential connection typical of USB ports and a wiring detection circuit which allows the micro to know when a computer has been connected.

13. The programmable, portable digital transducer device with high discrimination at low frequency and low intensity according to claim 1, **characterised in that** it comprises a case or casing (23) made from non-ferromagnetic material, wherein the circuitry is integrated on a printed circuit board or PCB, having been provided that it includes a special compartment (24) for housing a pair of primary lithium batteries (9) of 3V each, two LED indicators (15), a red LED and another green LED, on its upper front part whereby the type of programme and operation of the device is indicated, as well as a dual-colour LED indicator (25) which allows knowing the state of charge of the rechargeable internal battery when it is in the process of charging.

14. The programmable, portable digital transducer device with high discrimination at low frequency and low intensity according to claim 13, **characterised in that** together with the LED charge indicator the equipment includes a hole where the connector of the charger (26) is installed, at the other end or right side of the case there is located the Mini USB connector (12) whereby the equipment is programmed, while on the upper left side of the case there is located the push button (17) which allows turning the equipment on and off as well as starting or ending the programmed treatment:

15. The programmable, portable digital transducer device with high discrimination at low frequency and low intensity according to claim 13, **characterised in that** on the upper right side of the case there is located a female SMA connector used for screwing the male SMA connector used by the applicator.

16. The programmable, portable digital transducer device with high discrimination at low frequency and low intensity according to claim 1, **characterised in that** it includes electrostatic protection for the USB port, an internal reset button (27), a connector (28) with the signals of the JTAG or programming port of the microcontroller for updating the low level software, three jack type connectors whereby other ports or outputs and inputs of the microcontroller used can be programmed for the purpose of adding liquid crystal devices and manual control push buttons.

## Amended claims

### Amended claims under Art. 19.1 PCT

1. A programmable, portable digital transducer device with high discrimination at low frequency and low intensity, being of the type intended for generating electromagnetic fields for medical applications, **characterised in that** it is formed from small portable electronic equipment, powered by rechargeable or primary batteries (8-9), wherein there are established an intelligent battery charging circuit (1), a power supply circuit (2), a digital circuit (3), an analogue circuit (4), a USB connection and protection circuit (5), and optionally a liquid crystal display (6), having been provided that the power supply circuit incorporates generating means for generating three working voltages, one for the digital circuit (3) and two for the analogue circuit (4), while the digital circuit (3) is formed by an ARM microcontroller (7) which has at least one oscillator circuit (10-10'), preferably two, internally including a digital-analogue converter circuit (11) which allows generating any type of wave signals referenced to 0 volts through complementary programming software.

2. The programmable, portable digital transducer device with high discrimination at low frequency and low intensity according to claim 1, **characterised in that** the supply voltage of the digital circuit (3) is obtained using an LDO or fixed-voltage regulator, while the supply voltages of the analogue circuit are generated internally in the equipment using two different power supplies including respective integrated circuits.

3. The programmable, portable digital transducer device with high discrimination at low frequency and low intensity according to claim 1, **characterised in that** the two power supplies of the analogue circuit include a circuit allowing them to be turned on or turned off using an I/O of the microcontroller (7).

4. The programmable, portable digital transducer device with high discrimination at low frequency and low intensity according to claim 1, **characterised in that** the microcontroller (7) internally includes a pair of ports (18-19) and the firmware necessary for implementing a USB 2.0 connection (12).

5. The programmable, portable digital transducer device with high discrimination at low frequency and low intensity according to claim 1, **characterised in that** the analogue-digital converter (11) is included inside the microcontroller together with the USB connection and protection circuit (5) and a voltage splitter (14).

6. The programmable, portable digital transducer device with high discrimination at low frequency and low intensity according to claim 1, **characterised in that** the digital circuit includes control for the human machine interface consisting of the following devices:
- LED indicators (15).
- Buzzer or noise or tone generator (16).
- Liquid crystal display (6).
- On/off button of the equipment (17).
- Detection device for detecting a connection of an external computer to the USB port (12).
- Device for activating and disconnecting the signal generating circuit.
- Detection device for detecting the opening of the magnetic signal generating circuit.

7. The programmable, portable digital transducer device with high discrimination at low frequency and low intensity according to claim 1, **characterised in that** the digital circuit (3) includes a port which allows connecting the liquid crystal display (6) to the microcontroller if such device is necessary.

8. The programmable, portable digital transducer device with high discrimination at low frequency and low intensity according to claim 1, **characterised in that** the digital circuit (3) includes a multifunction button whereby the device can be turned on and off, and the treatment can be activated and deactivated.

9. The programmable, portable digital transducer device with high discrimination at low frequency and low intensity according to claim 1, **characterised in that** the analogue circuit (4) incorporates means for transforming the signals received in the form of voltage into NRO (Non Return to zero) signals converted into current signals with a maximum capacity of 1 milliampere, being able to be additionally set to a lower intensity by means of a software setting reducing the amplitude of the different generated signals in the digital-analogue converter which can also change the frequency and the working cycle of the generated signals.

10. The programmable; portable digital transducer device with high discrimination at low frequency and low intensity according to claim 1, **characterised in that** the analogue circuit (4) includes a continuity detecting circuit (20) keeping the microcontroller (7) informed about whether or not the coils (21) are connected to the equipment in a manner such that the working session can or cannot be started.

11. The programmable, portable digital transducer device with high discrimination at low frequency and low intensity according to claim 1, **characterised in that** the analogue circuit (4) includes an offset setting circuit (22).

12. The programmable, portable digital transducer device with high discrimination at low frequency and low intensity according to claim 1, **characterised in that** the USB connection and protection circuit (5) includes an integrated static protector, a filter circuit for the differential connection typical of USB ports and a wiring detection circuit which allows the micro to know when a computer has been connected.

13. The programmable, portable digital transducer device with high discrimination at low frequency and low intensity according to claim 1, **characterised in that** it comprises a case or casing (23) made from non-ferromagnetic material, wherein the circuitry is integrated on a printed circuit board or PCB, having been provided that it includes a special compartment (24) for housing a pair of primary lithium batteries (9) 3V each, two LED indicators (15), a red LED and another green LED, on its upper front part whereby the type of programme and operation of the device are indicated, as well as a dual-colour LED indicator (25) which allows knowing the state of charge of the rechargeable internal battery when it is in the process of charging.

14. The programmable, portable digital transducer device with high discrimination at low frequency and low intensity according to claim 13, **characterised in that** together with the LED charge indicator the equipment includes a hole where the connector of the charger (26) is installed, at the other end or right side of the case there is located the Mini USB connector (12) whereby the equipment is programmed, while on the upper left side of the case there is located the push button (17) which allows turning the equipment on and off as well as starting or ending the programmed treatment.

15. The programmable, portable digital transducer device with high discrimination at low frequency and low intensity, according to claim. 13, **characterised in that** on the upper right side of the case there is located a female SMA connector used for screwing the male SMA connector used by the applicator.

16. The programmable, portable digital transducer device with high discrimination at low frequency and low intensity according to claim 1, **characterised in that** it includes electrostatic protection for the USB port, an internal reset button (27), a connector (28) with the signals of the JTAG or programming port of the microcontroller for updating the low level software, three jack type connectors whereby other ports or outputs and inputs of the microcontroller used can be programmed for the purpose of adding liquid crystal devices and manual control push buttons
